# EUROPEAN PATENT SPECIFICATION

(11) **EP 2 831 265 B1**
(45) Date of publication and mention of the grant of the patent: **27.07.2016**
(21) Application number: 13712804.7
(22) Date of filing: 28.03.2013
(51) Int. Cl.: C12Q 1/24, C12M 1/00, C12N 11/00, B01L 3/00, C12M 3/00

(54) **METHODS FOR OBSERVING CELLS WITH CELL WALL OR INVERTEBRATE EMBRYOS WITH OBLONG EGGSHELL**
VERFAHREN ZUR BEOBACHTUNG VON ZELLEN MIT ZELLWAND ODER VON EMBRYONEN MIT LÄNGLICHER EIERSCHALE
PROCÉDÉS POUR OBSERVER DES CELLULES À PAROI CELLULAIRE OU DES EMBRYONS D'INVERTÉBRÉS À COQUILLE OBLONGUE

(30) Priority: 29.03.2012 EP 12305364
(43) Date of publication of application: 04.02.2015
(73) Proprietor: Centre National de la Recherche Scientifique, 75016 Paris (FR); Université de Strasbourg, 67000 Strasbourg (FR); INSERM - Institut National de la Santé et de la Recherche Médicale, 75013 Paris (FR)
(72) Inventor: RIVELINE, Daniel, F-67000 Strasbourg (FR)
(74) Representative: Lebrette, Camille
(86) International application number: PCT/EP2013/056721
(87) International publication number: WO 2013/144302

(56) References cited:
- WO-A1-2010/092116
- WO-A1-2010/142954
- US-A- 5 310 674
- US-A1- 2009 098 541
- US-A1- 2010 221 768
- US-B1- 6 538 810
- M DEUTCH ET AL: "Apparatus for high-precision repetitive sequential optical measurement of living cells", CYTOMETRY, vol. 16, 1 January 1994 (1994-01-01), pages 214-226, XP055032126,
- SEILA SELIMOVIC ET AL: "Microfabricated polyester conical microwells for cell culture applications", LAB ON A CHIP, vol. 11, no. 14, 1 January 2011 (2011-01-01), page 2325, XP055032136, ISSN: 1473-0197, DOI: 10.1039/c1lc20213h
- K. D. YOUNG: 'The Selective Value of Bacterial Shape' MICROBIOLOGY AND MOLECULAR BIOLOGY REVIEWS vol. 70, no. 3, 01 September 2006, US, pages 660 - 703, XP055246675 DOI: 10.1128/MMBR.00001-06 ISSN: 1092-2172
- PATRICIA A MAURICE ET AL: 'Direct observations of aluminosilicate weathering in the hyporheic zone of an Antarctic Dry Valley stream' GEOCHEMICA ET COSMOCHIMA ACTA vol. 66, no. 8, 01 January 2002, pages 1335 - 1347, XP055248854

## Description

### FIELD OF THE INVENTION

The present invention relates to methods for observing cells having a cell wall or invertebrate embryos with oblong eggs. It relates to the field of biology, pharmacology and diagnosis.

### BACKGROUND OF THE INVENTION

Bacteria and fungi are simple unicellular organisms very useful to study biological functions. For instance and non-exhaustively, the cytokinetic ring is a fundamental structure which causes the separation of cells at the end of mitosis. Cytokinetic ring dysfunction is a typical target for antitumor agents. Therefore, any device for studying such cells is very useful at a large-scale. In particular, as these cells are often oblong, the device has to be suitable for orienting the cells so as to obtain the same orientation for all cells and an orientation appropriate for the optimal observation of structures such as the cytokinetic ring.

Patent application WO 2010/092116 discloses such devices allowing the observation of the whole cytokinetic ring in the unique plane of closure and methods for using them and preparing them.

In addition, this problem is not specific to bacteria and fungi. It also exists for studying invertebrate embryos having an oblong eggshell such embryos of *Caenorhabditis elegans* and *Drosophila.* Indeed, the vertical orientation of embryos is often seeked.

For instance, the *Caenorhabditis elegans* embryo is particular amenable to microscopy and embryological studies because of its short developmental time, transparent shell and non-pigmented cells. Methods for mounting embryos such as poly-L-lysine-coated slides with grease feet (Mohler and Isaacson, 2010, Cold Spring Harb Protoc doi:10:1101/pdb.prot5388) or polymer beads result in random embryo orientation, which complicates analysis of development. In order to study *C*. *Elegans* embryos, means allowing consistent orientation such that every embryo is positioned in the same way should be required. Solutions have already proposed. For instance, Walston and Hardin (2010, Cold Spring Harb Protoc, prot5540) disclose a protocol in which the embryos are embedded in agar. However, this method results in slight compression of embryos.

Similarly, *Drosophilia,* especially *Drosophilia melanogaster,* is an important and convenient model. Its small size, short generation time, and large brood size make this embryo ideal for genetic studies. Transparent embryos facilitate developmental studies. The orientation of the *Drosophilia* embryos is very important for embryogenesis because of the interest of the dorsoventral axis of the embryos. When studied on a regular microscope slide, the dorsoventral orientation is essentially random, preventing high-throughput analysis. Chung et al (2011, Nature Methods, 8, 171-177) discloses a microfluidic array for large-scale ordering and orientation of embryos. However, the array disclosed is very complex, expensive and not easily available for the researcher.

Therefore, devices and methods allowing to provide the appropriate orientation to a high number of cells with cell wall or embryos are still needed.

### SUMMARY

In the present document, it is provided improved devices (not claimed) for observing cells and embryos having a rigid oblong wall, namely cells with a cell wall or invertebrate embryos having an oblong eggshell. These devices provide a very high rate of filling in of wells. The improvement is based on wells with conical or frustoconical shape. Indeed, in the same conditions and for cells with cell wall, the improved devices of the invention show 80 % of filling in whereas the devices with wells of cylindrical shape show only 20 %. In addition, the devices of the document allow to obtain immobilization of a plurality of cells or embryos with the same orientation, namely with a long axis parallel to the observation plane, First at all, this orientation may allow an optimal observation of cellular structure, such as the cytokinetic ring which is then perpendicular to the observation plane. Secondly, through the benefit of having a plurality of cells or embryos with the same orientation, it allows obtaining data which are easily comparable. It is particularly interesting for *Drosophilia* embryos because the dorsoventral orientation is perpendicular to the observation plane.

Accordingly, the present document discloses a device for immobilizing on a support, with the same orientation, a plurality of cells having cell wall or embryos having eggshell, said cells or embryos having an oblong shape, wherein the device comprises a support bearing a plurality of wells suitable for containing only one single cell or embryo, characterized in that:
- the wells have a conical or frustoconical shape;
- the width of the top of the wells is greater than the short axis of the cells or of the embryos to be immobilized in the wells; and
- the width of the bottom of the wells is smaller than the short axis of the cells or of the embryos to be immobilized in the wells.

The claimed invention generally relates to a method for immobilizing on a support of a device a plurality of cells or invertebrate embryos with the same orientation, as disclosed in claim 1.

Subject-matter defined hereinafter as the "invention" or "embodiment" or "aspect" etc that is broader in scope than claim 1 (e.g. devices) does not constitute the claimed invention, rather background art for improved understanding. Optionally, the method further comprises a previous step of selecting a suitable device for immobilizing the cells or embryos, in particular based on the cells or embryos size, more particular their the short axis.

Preferably, the step of placing the cells or embryos into the wells is carried out by a centrifugation step.

Preferably, the method comprises a step of observing the cells or embryos immobilized on the support, in particular in a perpendicular plane to the long axis of cells or of embryos.

Preferably, the width of wells equal to the short axis of the cells or embryos to be immobilized in the wells is located in the lower half of the wells, preferably in the lower third of the wells.

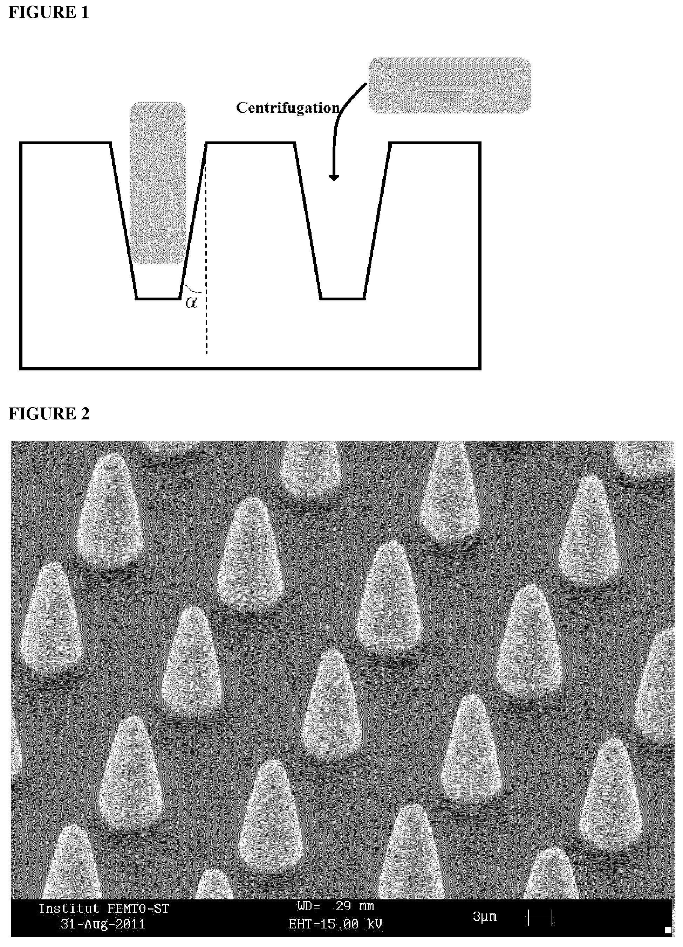

Preferably, the angle (α as shown in Figure 1) between the vertical axis of the wells and the side of the wells is between 5 and 45°, preferably between 10 and 30°.

Preferably, the depth of the wells is at least 1/3 of the long axis of the cells or embryos to be immobilized in the wells, preferably at least or about 1/2 of the long axis of the cells or embryos to be immobilized in the wells.

In a preferred embodiment, the cells are yeast or bacteria and the embryos are embryos of *C*. *elegans* or *Drosophila.*

In a particular embodiment, the cells are yeast, the cells are yeast and the width of the top of the wells is between 6 to 10 µm and the width of the bottom of the wells is less than 1-2 µm. Optionally, the wells depth is between 4 to 10 µm, preferably between 6 to 8 µm.

In another particular embodiment, the cells are bacteria and the width of the top of the wells is comprised between 500 nm and 3 µm, and the width of the bottom of the wells is less than 500 nm.

In another particular embodiment, the embryos are embryos of *C*. *Elegans* and the width of the top of the wells is comprised between 26 µm and 40 µm, and the width of the bottom of the wells is less than 25 µm. Optionally the well depth is between 10 and 40 µm

In a further particular embodiment, the embryos are embryos of *Drosophila* and the width of the top of the wells is comprised between 0.2 mm and 0.3 mm, and the width of the bottom of the wells is less than 0.16 mm. Optionally the well depth is between 0.15 and 0.5 mm.

Preferably, the support is suitable for microscopy. In particular, the support is a plate of glass, preferably a silanised glass, more preferably a silanised glass coverslip, with microfabricated substrate bearing the wells. However, a quartz coverslip is also considered because it allows a high resolution.

Preferably, the microfabricated substrate is made of poly(dimethylsiloxane) (PDMS). Optionally, the device further comprises physical barriers separating several groups of wells from each other on the device. In addition, such a device can further comprise a microfluidic system in order to address different fluids (e.g., samples or media) to the groups of wells on the device.

Optionally, the device comprises several sets of wells, each set having a different width at the top of the wells.

The present invention concerns a method for observing or studying cells or embryos, comprising a) providing a device by performing the method of the present invention; and b) observing the cells or embryos, in particular in a perpendicular plane to the long axis of cells or of embryos. Preferably, the observation is carried out by microscopy.

Then, according to the method of the present invention, the step of observing or studying the cells or embryos may comprise:
- observing the cytokinetic ring of said cells, and/or
- screening or identifying a test molecule able to modulate the cell division of said cells or the embryogenesis of said embryos, and/or
- observing a plane perpendicular to the long axis of said cells or of said embryos, and/or
- screening or identifying a test molecule able to modulate the tip growth of cells, and/or
- determining the level of expression or activity of proteins in said cells or embryos, and/or
- determining the localization or the interaction of proteins in said cells or embryos, and/or
- observing the structure, localization, shape and/or integrity of organelles, cytoskeleton, DNA, or cytokinetic ring in said cells or embryos, and/or
- observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring in said cells or embryos.

The present invention further concerns the use of a device according to the present invention for observing or studying cells or embryos, in particular for observing the cytokinetic ring of cells, for screening or identifying a test molecule able to modulate the cell division or the embryogenesis, for observing a plane perpendicular to the long axis of cells or of embryos, for screening or identifying a test molecule able to modulate the tip growth of cells, for determining the level of expression or activity of proteins, for determining the localization or the interaction of proteins, for observing the structure, localization, shape and/or integrity of organelles, cytoskeleton, DNA, or cytokinetic ring, or for observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring.

### BRIEF DESCRIPTION OF THE DRAWINGS

**Figure 1****:** Schematic drawing of the wells. Cells or embryos are inserted into conical shapes cavities (by centrifugation for example).
**Figure 2****:** Conical molds. Masters were prepared on silicone wafers by standard microfabrication methods.
**Figure 3****:** Fillings of wells with fission yeasts. In inset, rings are shown (Scale bar, 5 µm).
**Figure 4****:** Other cells can be cultured in the same set-up with conical cavities, here *S*. *cerevisiae.* Their cycle time is unchanged (Scale bar, 10 µm).
**Figure 5****:** *C.elegans* in egg cups. Fig 5a) C. *elegans* embryo on a flat glass surface (left) and in an egg cup (right). Fig 5b) Time lapse of the developing embryo in the egg cups. Its characteristic movement and contractions are visible. Fig 5c) The contour of the worm is drawn to emphasize its rapid movements. (scale bars = 10µm).

### DESCRIPTION

In the present document, the inventors provide devices (not claimed) and methods using them designed to be used with cells having a cell wall and an oblong shape or with invertebrate embryos having an oblong eggshell.

In particular, the devices are improved because of the conical or frustoconical shape of the wells, allowing a dramatic increase of the filling in of the wells and the control of the cell or embryo orientation. Indeed, without being linked by a theory, the conical or frustoconical shape of the wells facilitates the entry of the cells or embryos into the wells, but also increases their capture into the wells. The conical or frustoconical shape of the wells is suitable for allowing cells and embryos to resist to hydrodynamic flow, for instance during washing steps. In addition, they present the advantages of limiting the compression of cells or embryos. The other advantage is that, by selecting the appropriate size of wells for each cell or embryo and due to their oblong shape, the device allows the same orientation of cells or embryos, in particular with the long axis thereof perpendicular to the observation plane or the support. Accordingly, the device allows the study of a large number of cells or embryos, having the same orientation, said orientation being suitable for observing any plane perpendicular to the long axis of cells or embryos.

Accordingly, the present document discloses a device comprising a support bearing a plurality of wells suitable for containing only one single cell or embryo, characterized in that wells have a conical or frustoconical shape. In a preferred embodiment, wells have a frustoconical shape. In another preferred embodiment, wells have a conical shape.

The present invention generally concerns a method as recited in claim 1.

The basis of the conical or frustoconical shape is preferably a circle. However, the present invention also contemplates the conical or frustoconical shapes with a square basis (i.e., pyramidal or frustopyramidal shape) or with a polygonal basis, providing that the summits of the polygon are placed on a circle. Preferably, the conical or frustoconical shape has an axis passing through the centre of the basis at right angles to its plane. By "frustoconical" is intended that the apex of the conical shape is truncated, the truncation plane being essentially parallel to the cone's base.

By "for containing only one single cell" is intended that the well is suitable for containing one and only one cell. By "for containing only one single embryo" is intended that the well is suitable for containing one and only one embryo. By containing only one single cell or embryo is intended that the size of the wells makes it suitable for containing only one cell or embryo to be immobilized in the wells, namely the wells being too small to contain two cells or embryos.

By "for orienting the long axis perpendicular to the observation plane" is intended that the structure of the well is adapted to obtain the suitable orientation of the cells or embryos. Indeed, cells and embryos are rigid due to the cell wall or the eggshell and have an oblong shape. Accordingly, one can define a short axis and a long axis for the cell or embryo. By "oblong" is intended that the cell or embryo has a long axis being at least twice of the short axis. Cells or embryos have to be placed so that the long axis is perpendicular to the support. Then, a well having a width smaller than the long axis allows the intended orientation of cells or embryos.

The shape of the wells may for instance be defined by the width of the top of the wells and the width of the bottom of the wells. By "top" is intended the upper part of the well comprising the opening of the well. By "bottom" is intended the lower part of the well, opposite to the top. In a most preferred embodiment, the bottom of the well is closed.

Accordingly, the width of the top of the wells is greater than the short axis of cells or embryos to be immobilized in the wells. In particular, the width of the top of the wells may be for instance at least 10, 15, 20, 25 or 30 % greater, preferably about 10, 15, 20, 25 or 30 % greater. In addition, the width of the bottom of the wells is smaller than the short axis of cells or embryos. In particular, the width of the bottom of the wells may be at least 10, 15, 20, 25 or 30 % smaller, preferably about 10, 15, 20, 25 or 30 % smaller. By the term "about" is intended to mean the value more or less 5 % thereof.

According to the conical or frustoconical shape of the wells, the lateral sides of wells form an angle with the vertical axis of the well (α as shown in Figure 1). This angle between the vertical axis of the well and the side of the well is between 5 and 45°, preferably between 10 and 30°. The vertical axis is the axis which is perpendicular to the opening of the well.

In addition, another parameter should be the depth of the wells. The depth of the well is the distance between the top and the bottom of the well. The depth of wells is such that it prevents cells to move away when subjected to an external flow, and it orients the closure plane parallel to the observation plane. In a preferred embodiment, the depth of wells is preferably at least or about 1/3 of the long axis of cells or embryos to be immobilized in the wells, preferably at least or about 1/2. By "at least or about" is intended at least 1/3 or 1/2 of the long axis of cells or embryos or 1/3 or 1/2 of the long axis of cells or embryos, more or less 5 % thereof. For instance, it can be comprised between 1/3 of the long axis of cells or embryos and 3/2 of the long axis, more preferably between 1/2 of the long axis of cells or embryos and the full length of the long axis. The depth of each well can also be almost or about the long axis, thereby the whole cell or embryo is contained into the well. However, a well depth between 1/2 and 3/4 of the long axis of cells or embryos is preferred. One can also define the depth of the wells by a ratio to the width of the wells. Accordingly, the depth of the wells is at least 2/3 of the width of the top of the wells, preferably at least the width of the top of the wells. More preferably, the depth of the wells is almost or about twice the width of the top of the wells.

Another parameter is the location, in the wells, of the well width corresponding to the short axis of the cell or embryo to be immobilized. Preferably, the width of the well equal (or corresponding) to the short axis of the cell or embryo is located in the lower half of the well, preferably in the lower third of the well.

When cells are bacteria, the short axis is comprised between 0.5 and 3 µm and the long axis (the length) is quite variable. In case of bacteria having generally a short axis comprised between 0.5 and 3 µm, the well width at the top can be comprised between 500 nm and 3 µm. For instance, a convenient well for number of bacteria may have a well width at the top greater than 3 µm (e.g., 4-5 µm) and a width at the bottom of less than 500 nm (e.g., 0-400 nm). Wells with those features are adapted for most of the bacteria. The long axis of bacteria is highly variable among bacteria. However, for instance, a depth of 1, 2, 3, 5, 7 or 10 µm can be appropriate. In particular for E. coli, considering that the short axis is about 800 nm, the well width at the top is greater than 800 nm whereas the width at the bottom is smaller than 800 nm. For instance, the width at the top can be between 850 nm and 1 µm whereas the width at the bottom can be less than 750 nm, for instance between 500 to 750 nm or less. The depth can be 2 µm. In a preferred embodiment, the bacteria are rod-shaped bacteria, in particular bacilli.

When cells are fungi, they are preferably yeast. Typically, yeasts may have an oblong shape. The diameter or short axis of yeast is between 1 to 6 µm, more typically between 3 to 5 µm. Therefore, the well width at the top is greater than the diameter or the short axis whereas the width at the bottom is smaller than the diameter or the short axis. For instance, convenient wells may have a well width at the top of about 6-10 µm and a width at the bottom is less than 1-2 µm. Wells with those features are adapted for most of the yeast. Optionally, the wells depth is between 4 to 10 µm, preferably between 6 to 8 µm.

An interesting parameter to study in bacteria and yeast is the cytokinetic ring, and in particular the closure of the ring. Therefore, an advantage of the device according to the invention is that the bacteria and yeast are immobilized on the support so as the closure plane of the ring is parallel to the observation plane. By "the closure plane of the ring parallel to the observation plane" is intended that the closure plane of the cytokinetic ring is parallel to the support. The observation or focal plane is defined herein, where the structure of interest, i.e. the ring, appears entirely on the image. The direction along the wells depth is perpendicular to this plane.

For cells having a cell wall such as bacteria and yeast, generally having oblong or rod shape, the cytokinetic ring is always perpendicular to the long axis. Accordingly, the orientation of the cytokinetic ring can easily be controlled through the appropriate orientation of cells in the device. The same set-up will work also for spherical cells with walls such as S. *cerevisiae,* since they elongate during growth. For bacteria and fungal cells, an appropriate placement of the cells is sufficient to obtain the desired orientation of the closure plane of the ring.

The cell suitable for being used with the device can be any eukaryotic or prokaryotic cell having a cell wall. In particular, prokaryotic cells can be bacteria and eukaryotic cells can be a fungal cell such as yeast. The cell can be wild type, genetically engineered cells (mutated or recombinant cells) or cells treated with a molecule.

The well size (i.e., width and depth) can be adapted easily by the man skilled in the art for each specific cell to study. Optionally, the device comprises several sets of wells, each set having a different width. For instance, the well may have a conical shape and the different sets of wells may have well widths at the top selected from the group consisting of 10, 9, 8, 7, 6, 5, 4, 3, 2 and 1 gm. The depth of the different set of wells may be the same or different. The depth may be selected from the group consisting of 4, 5, 6, 7, 8, 9 and 10 µm.

In another embodiment, the wells are suitable to observe invertebrate embryos having an oblong eggshell.

In a first particular embodiment, the embryos are *Drosophila* embryos. Drosophila embryos have typically an oblong shape with a short axis of 0.16 to 0.21 mm and a long axis of 0.44 to 0.57 mm. Accordingly, the wells may have a width at the top of the wells of more than 0.16 mm, preferably more than 0.21 mm, and a width at the bottom of the wells of less than 0.16 mm. For instance, the width at the top may be of 0.2 to 0.3 mm whereas the width at the bottom is less than 0.16 mm, for instance less than 0.1 mm, more preferably between 0 and 0.1 mm. The depth of the wells is at least 0.15 mm and less than 1 mm. More preferably, the depth is between 0.15 and 0.5 mm.

In a second particular embodiment, the embryos are worm or nematode embryos, in particular *C*. *elegans* embryos. *C*. *elegans* embryos have typically an oblong shape with a short axis of 25 µm and a long axis of 40 µm. Accordingly, the wells may have a width at the top of the wells of more than 25 µm, and a width at the bottom of the wells of less than 25 µm. For instance, the width at the top may be between 26 µm and 40 µm whereas the width at the bottom is less than 20 µm, more preferably between 0 and 20 µm. The depth of the wells is at least 10 µm and less than 60 µm. More preferably, the depth is between 10 and 40 µm.

The wells with the conical or frustoconical shape can further be combined with additional structural elements. For instance, at the basis or bottom of wells, other elements can be added such as channels. Similarly, the wells having the conical or frustoconical shape can further include grooves in their walls (i.e., interior surface of wells), preferably grooves elongated from the top to the bottom. Such groves may allow culture medium circulation.

The support preferably comprises a plate and a microfabricated substrate.

Preferably, the microfabricated substrate of the support comprises a high number of wells, e.g., at least or about 10, 20, 50, 100, 150, 200, 300, 400, 500, 1,000, 5,000, 10,000, 50,000, 100,000, 500,000, or 1,000,000 wells. For instance, the support comprises at least 100 wells per cm² of substrate, preferably at least 5,000 wells by cm² of substrate, more preferably at least 10,000 wells by cm² of substrate. The wells are sufficiently separated to be discriminated during microscopy observation. In a particular embodiment, the wells are spaced of at least 200 nm, preferably by about 200 nm, 500 nm, 1 µm, 5 µm, or 10 µm. The space between the wells can optionally be treated with a cytophobic material (i.e., preventing cell adhesion), for instance polyethyleneglycol (PEG). The wells of the microfabricated substrate can have a bottom made of the microfabricated substrate or can go through the microfabricated substrate.

By "microfabricated substrate" is intended a microfabricated solid surface including, e.g., silicon, functionalized glass, germanium, ceramic, a semiconductor material, PTFE, carbon, polycarbonate, mica, mylar, plastic, quartz, polystyrene, gallium arsenide, gold, silver, metal, metal alloy, fabric, and combinations thereof. In particular, the microfabricated substrate is made of a solid material preferably biocompatible or with a surface treatment that makes it biocompatible. The following materials can be used for microfabrication: a polymer that can be crosslinked (PDMS, agar, polyacrylamide (PAA)...), a glassy material (polycarbonate (PC), polystyrene (PS)...), a metal or a semiconductor material.

The support has to be convenient for confocal, optical and/or fluorescence microscopies. An appropriate support can be any flat substrate (i.e., the plate) promoting a good adhesion with the polymer used for the microstructure (i.e., the microfabricated substrate). In a more preferred embodiment, the plate comprises or is a plate of glass. For example, a convenient plate according to the present invention is a coverslip or a slide. In a preferred embodiment, the coverslip is as thin as possible. For instance, the thickness of 0.085 to 0.13 mm is convenient.

Microfabrication techniques for preparing microfabricated substrates are well-known by the man skilled in the art.

For instance, microfabrication techniques for preparing the stamp used to produce the microfabricated substrate of the device can be for instance photolithography, thin-film deposition, wet chemical etching, reactive ion etching, inductively coupled plasma deep silicon etching, laser ablation, air abrasion techniques, and other techniques. Polymeric substrate materials are preferred for their ease of manufacture, low cost and disposability, as well as their general inertness. The polymeric substrate materials are preferably cross-linkable. They can be biocompatible and have a weak adhesion for cells. In a preferred embodiment, the substrate material is made of PDMS. Techniques are disclosed for instance in the following patents US 6,753,131; US 6,516,168 and US 6,143,412.

The substrate materials for the stamp can comprise the following polymeric materials without to be limited thereto:
- glassy polymers, such as polystyrene, polymethylmethacrylate (PMMA), polycarbonate, polytetrafluoroethylene (TEFLON®), polyvinylchloride (PVC);
- elastomeric materials polydimethylsiloxane (PDMS), polybutadiene, polyurethane, and the like;
- gels (agar, PAA...) and the like.

The microfabricated substrates of the device are readily manufactured from masters, using well-known molding techniques, such as injection molding, hot embossing or by molding a polymer that might be crosslinked (soft lithography). The substrate materials for the microfabricated substrates of the device can be the same than stamps and can comprise the following polymeric materials without to be limited thereto:
- glassy polymers, such as polystyrene, polymethylmethacrylate (PMMA), polycarbonate, polytetrafluoroethylene (TEFLON®), polyvinylchloride (PVC);
- elastomeric materials polydimethylsiloxane (PDMS), polybutadiene, polyurethane, and the like;
- gels (agar, PAA...) and the like, with the advantage of allowing the diffusion of nutrients within the microfabricated structure and therefore the nutrients can diffuse through the side all around the wells.

In a preferred embodiment, the microfabricated substrate is made of biocompatible materials and has a weak adherence for cells. In a most preferred embodiment, the microfabricated substrate is made of PDMS. In a second preferred embodiment, the microfabricated substrate is made of gel including agar, PAA (poly acrylamide) and the like.

In a particular embodiment, the resulting microfabricated substrate has for instance a thickness of about 20-100 µm, preferably about 30-50 µm. Preferably, the siloxane-based polymer is PDMS. Preferably, the support is a glass coverslip, more preferably as thin as possible.

For instance in the case of the cytokinetic ring, due to the working distance and the resolution needed to observe it, the thickness of the support need to be as thin as possible. Accordingly, the thickness is preferably less than 200 µm, more preferably less than 150 µm, still more preferably between 100 and 150 µm. However, depending on where is placed the objective, the thickness is not an issue. Indeed, the objective may be placed under the cell or embryo to be observed, and there is no more thickness constraint.

Accordingly, in a first particular preferred embodiment, the device comprises a support bearing a plurality of wells suitable for containing a unique cell or embryo as defined above. The support comprises a plate and a microfabricated substrate supported by the plate. The plate is preferably a glass coverslip. The microfabricated substrate is supported by the coverslip. The microfabricated substrate is for instance PDMS substrate or gel. The nutrients can be added by the upper side of the wells.

The device of the present invention is useful for studying the cytokinetic ring of bacteria or yeast. Of course, the observation of the ring can be combined with other parameters of interest. The cytokinetic ring can be observed by microscopy, in particular fluorescence microscopy. Generally, the cytokinetic ring is observed through fluorescent proteins (Glotzer M, Science. 2005 Mar 18;307(5716):1735-9).

The fluorescent proteins can be any protein of the cytokinetic ring. For instance for fission yeast, the fluorescently labelled protein (e.g., by YFP, GFP, EGFP, mCherry and the like) can be myosin (e.g., myosin II regulatory light chain such as RLC1-mCherry, or myosin II heavy chain such as myo2-YFP), paxillin-like protein Px11 (such as px11-GFP), membrane-cytoskeletal interactions (e.g., F-BAR domains such as cdc15-YFP), 1,3-beta-glucan synthase (e.g., catalytic subunit Bgs1 such as bgs1-GFP) and the like. Other proteins suitable are disclosed in the articles of Wu and Pollard (Wu et al, Methods Cell Biol. 2008;89:253-73; Vavylonis et al, Science. 2008 Jan 4;319(5859):97-100 ; Wu et al, J Cell Biol. 2006 Jul 31;174(3):391-402; Wu and Pollard, Science. 2005 Oct 14;310(5746):310-4.; Kovar et al, Mol Biol Cell. 2005 May;16(5):2313-24 ; Wu et al, Dev Cell. 2003 Nov;5(5):723-34), and their homologs in other cells will be appropriate. In case of fluorescently labelled proteins, the cells are genetically engineered in order to express such fluorescently labelled proteins. Alternatively, the ring can be observed by using fluorescently labelled ring marker or antibody directed against any protein of the ring. For instance, rhodamine or Alexa- phalloidine is suitable for this use, as well as immunofluorescence staining with anti-myosin antibodies (Glotzer M, Science. 2005 Mar 18;307(5716):1735-9).

The present invention further concerns a method for observing or studying cells or embryos, comprising a) providing a device performing the method of the present invention; and b) observing the cells or embryos, in particular in a perpendicular plane to the long axis of cells or of embryos. It also concerns the use of a method according to the present invention for observing or studying cells or embryos by microscopy, and in particular in any plane perpendicular to the long axis of cells or of embryos. Cells and embryos are as defined above. Indeed, the method of the invention is well appropriate for high-throughput multi-parameters studies.

The step a) may comprise providing the cells or embryos to be studied or observed; selecting a device adapted for the provided cells or embryos and placing the cells on the device.

The method may comprise a step of loading the cells or embryos on the device of the present invention. The cells or embryos can be placed into the wells of the device by any means known in the art. For instance, the cells or embryos can be introduced in it by centrifugation, preferably followed by washing steps.

As explained above, the method allows the study of a high number of cells or embryos, all with the same orientation. The method also allows benefiting to a good observation plane for the structures for which there is an advantage to observe them in a plane perpendicular to the long axis, such as the cytokinetic ring for bacteria or yeast, the dorsoventral axis for *Drosophila* embryos or cytokinetic ring or cell-cell junctions for *C. elegans* embryos.

Accordingly, non-exhaustively, the method may be useful for:
- observing the cytokinetic ring of bacteria or yeast; the method being detailed below or disclosed in WO 2010/092116; and/or;
- observing the tip growth of cells with cell wall;
- for measuring the stress in a yeast cell by introducing a labeled tRNA into the cell and detecting a change in subcellular localization of the labeled tRNA in the cell (see, WO 2012/011110); and/or
- for observing the localization or the interaction of labelled proteins, molecules or nucleic acids in cells or embryos; and/or
- for observing the effect of a molecule, an antibody, a drug, or a siRNA on the embryogenesis with the invertebrate embryos; and/or
- for determining the level of expression of proteins; and/or,
- for determining the level of activity of proteins, and/or
- for observing the structure, localization, shape and/or integrity of organelles, cytoskeleton, DNA, or cytokinetic ring; and/or
- for observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring.

When the method comprises the step of determining the level of expression of proteins, the level of expression of proteins can be assessed by labelling the protein to be observed or by labelling the mRNA encoding this protein. The methods for labelling proteins or mRNA are well-known in the art. It can be directly labelled by conjugating the protein to a label such as a fluorescent label. Alternatively, it can be indirectly labelled by using antibody specific of the protein to be observed. The detection of mRNA may be carried out by using labelled probes specific of the mRNA to be detected. The level of expression is assessed through the measurement of the intensity of the labelling, preferably the fluorescent or radioactive labelling. The level of expression can be measured at one time or for a period of time. It can be measured for one protein or for several.

When the method comprises the step of determining the level of activity of a protein, the one skilled in the art can adapt the assay to measure the activity. For instance, in case of kinases or phosphatases, the activity can be assessed by the measurement of the amount of phosphorylated or unphosphorylated proteins which are the substrates of the kinases or phosphatases. For instance, antibodies specific for the phosphorylated or unphosphorylated form of a protein can be used. Other activities can be easily detected such as methylation, sumoylation, and the like. In case of enzymes, the appearance and disappearance of substrates or products can be assessed.

When the method comprises the step of determining the localization or the interaction of proteins, it can be observed by labelling the proteins, preferably by fluorescence, and, in case of the interaction, by using distinct labels for each interaction partners. In case of interaction, a set of fluorophores/quencher or a set of fluorophores allowing fluorescence transfer may be used.

When the method comprises the step of observing the structure, localization, shape and/or integrity of organelles, cytoskeleton, DNA, or cytokinetic ring, the methods for observing organelles, cytoskeleton, DNA, or cytokinetic ring are well known by the one skilled in the art. Organelles may be non-exhaustively selected from endoplasmic reticulum, nucleus, Golgi apparatus, mitochondria, centrioles and vacuole.

When the method comprises the step of observing the effect of molecules, antibodies, drugs, or siRNA on cells, in particular on the level of expression of proteins, the level of activity of proteins, the localization or interaction of proteins, the structure, localization, shape and/or integrity of organelles, cytoskeleton, DNA, or cytokinetic ring, the closure of the cytokinetic ring, it can be carried at one time or for a period of time. For instance, it can be useful for screening methods, or for methods for determining the efficiency or toxicity of drugs.

In a preferred embodiment of the present invention, a combination of these parameters is observed. Indeed, an advantage of the present method is to allow a multi-parametric analysis of cells. For instance, at least two, three or four of the above parameters may be combined. In addition, the study of these parameters through a period of time allows the analysis of the dynamics. In a very particular embodiment, the step of observing cells includes at least observing the cytokinetic ring, in particular its closure.

The invention also relates to a method of screening molecules of interest. Indeed, after contacting cells or embryos with candidate molecules, cells and embryos may be observed in order to identify differences. For instance, by observing the cell division, the method allows the selection of molecules modulating the division. In another example, by observing the embryogenesis, one can select or identify molecules having an impact on this phenomenon.

Accordingly, the present invention concerns a method for screening or identifying a molecule of interest able to modulate the cell division or embryogenesis, comprising:
a) providing a device according to the method of the present invention bearing cells or embryos which have been contacted with a test molecule;
b) assessing the cell division or the embryogenesis;
c) comparing the cell division or the embryogenesis in presence and in absence of the molecule; and,
d) selecting the molecule for which the cell division or the embryogenesis is significantly different in presence and in absence of the molecule, thereby identifying a molecule able to modulate the cell division or embryogenesis.

In particular, the step a) comprises providing cells or embryos on which the molecule has to be tested; selecting a device adapted for the provided cells or embryos and placing the cells or embryos on the device.

In a first embodiment, the cells or embryos are contacting with the molecule before being placed on the devices of the invention. Accordingly, the method can comprise previous steps of contacting cells or embryos with the molecule and loading the contacted cells or embryos on the device. In an alternative embodiment, the cells or embryos are contacting with the molecule when they are already placed on the devices.

Accordingly, the method can comprise previous steps of loading the cells or embryos on the device of the present invention and contacting cells or embryos with the test molecule. The advantage of the method of the present invention is that a high number of test molecules can be simultaneously assayed due to the high number of wells and the possible automated record.

In a more specific embodiment, the present invention concerns a method for observing the cytokinetic ring of cells, in particular of bacteria or yeast, comprising a) providing a device according to the method of the present invention bearing the cells; and b) observing the cytokinetic ring (i.e., with the closure plane of the cytokinetic ring parallel to the observation plane). In particular, the step a) comprises providing the cells for which the cytokinetic ring has to be observed; selecting a device adapted for the provided cells and placing the cells on the device. It also concerns the use of a method 2. according to the invention for observing the cytokinetic ring of cells with the closure plane of the cytokinetic ring parallel to the observation plane. In addition to the cytokinetic ring, the method may further include the observation of other organelles or proteins, as detailed above.

Observing the cytokinetic ring of bacteria or yeast may be useful in the screening of molecules of interest. Therefore, the present invention concerns the use of the method of the present invention for molecules screening. Indeed, the method of the present invention is used to assay or test the ability of a test molecule to modulate the cell division, in particular to assay or test the ability of a test molecule to modulate the closure of the ring. In particular, a molecule of interest is a molecule able to block or inhibit the cell division, in particular through the blockage or inhibition of the closure of the cytokinetic ring. Accordingly, the present invention concerns a method for screening or identifying a test molecule able to modulate the cell division comprising:
a) providing a device according to the method of the present invention bearing cells which have been contacted with a test molecule;
b) assessing the closure of the ring of the cells;
c) comparing the closure of the ring of the cells in presence and in absence of the test molecule; and,
d) selecting the test molecule for which the closure is significantly different in presence and in absence of the test molecule, thereby identifying a test molecule able to modulate the cell division.

Preferably, cells are bacteria or yeast. In particular, the step a) comprises providing cells on which the test molecule has to be tested; selecting a device adapted for the provided cells and placing the cells on the device.

In a first embodiment, the cells are contacting with the test molecule before being placed on the devices.

Accordingly, the method can comprise previous steps of contacting cells with the test molecule and loading the contacted cells on the device. In an alternative embodiment, the cells are contacting with the test molecule when they are already placed on the devices.

Accordingly, the method can comprise previous steps of loading the cells on the device and contacting cells with the test molecule.

The cells can be placed into the wells of the device by any means known in the art. For instance, the cells can be introduced into wells by centrifugation, preferably followed by washing steps.

In a first embodiment, the step of assessing the closure of the ring comprises the measure of the number of cells having an open ring and of the cells having a closed ring. In this embodiment, the closure is significantly different in the presence and in the absence of the test molecule when the number of open and closed ring is significantly different in presence and in absence of the test molecule. In a second embodiment, the step of assessing the closure of the ring comprises the measure of the velocity of the ring closure. In this embodiment, the closure is significantly different in presence and in absence of the test molecule when the velocity is significantly different in presence and in absence of the test molecule. In a third embodiment, the step of assessing the closure of the ring comprises both the measure of the number of cells having an opened ring and of the cells having a closed ring and the measure of the velocity of the ring closure. In a fourth embodiment, the step of assessing the closure of the ring comprises registering the cytokinetic ring for several cells either in presence or in absence of the test molecule. By "several" is preferably intended at least 100, 1,000, 10,000, 100,000 or 1,000,000 cells in each condition. If the test molecule does not modulate the cell division, the ring of cells can statistically have any position. The superposition of the registered rings results in a disk. Alternatively, if the test molecule blocks or inhibits the ring closure, the ring of cells has a higher probability to be opened. The superposition of the registered rings results in a circle. Therefore, a test molecule blocking or inhibiting the ring closure can be selected through the form for the superposition of the registered rings.

The cells can be synchronized at the beginning of the method or not.

When the cells are prokaryotic, the identified molecules have an interest as antibiotic agents and the method is a method for screening or identifying a test molecule having antibiotic property. When the cells are eukaryotic, the identified molecules have an interest as anti-proliferative agents and the method is a method for screening or identifying a test molecule having anti-proliferative property, in particular anti-tumor property. When the cells are fungi, the identified molecules have an interest as anti-fungal agents and the approach is a method for screening or identifying a test molecule having anti-fungal property.

The test molecule may be of various origin, nature and composition. It may be any organic or inorganic substance, such as a lipid, peptide, polypeptide, nucleic acid, small molecule, etc., isolated or mixed with other substances. For instance, the test compound can be an antibody, an antisense oligonucleotide, or an RNAi. The molecule may be all or part of a combinatorial library of products, for instance.

The advantage of the method of the present invention is that a high number of test molecules can be simultaneously assayed due to the high number of wells and the automated record of the ring closure. In this embodiment, the device can comprise several groups of wells on the same plate separated from each other such that each group can be incubated in a different medium. For instance, a group of wells can be contacted with a test molecule and another group can be contacted with another test molecule or without any test molecule. This separation can be provided by a physical barrier such as teflon seal or directly PDMS molded separations. For example, see SPI Teflon® of SPI Supplies. For instance, each group of wells can comprise at least or about 100, 1,000, 10,000, 100,000 or 1,000,000 wells. Microfluidic system can be further used in order to address a different drug at different locations or groups of wells on the device (Melin J, Quake SR., Annu Rev Biophys Biomol Struct. 2007;36:213-31 ; Hansen C, Quake SR, Curr Opin Struct Biol. 2003 Oct;13(5):538-44 ; Hong JW, Quake SR, Nat Biotechnol. 2003 Oct;21(10):1179-83 ; Quake SR, Scherer A, Science. 2000 Nov 24;290(5496):1536-40).

In addition to the observation of the cytokinetic ring, the method may also comprise a step of observing

In another embodiment and in the context of cells with a cell wall, the devices are also useful to observe any plane perpendicular to the long axis of the cells. Preferably, the cells are yeast. The tip growth of yeast is an interesting phenomenon for the scientific community but it cannot be easily observed with the available devices. Therefore, the device provides the means to conveniently observe the tip, and then the tip growth.

Therefore, the present invention also concerns a method for observing a plane perpendicular to a long axis of cells with cell wall and having an oval or rod shape, comprising a) providing a device according to the method of the present invention bearing the cells; and b) observing the plane parallel to the observation plane.

The present invention further concerns the use of a methods according to the present invention for observing a plane perpendicular to a long axis of cells with cell wall with a parallel arrangement to the observation plane. In a preferred embodiment, the observed perpendicular plane is the tip of the cells.

In addition, the present invention concerns the use of a method according to the present invention for screening or identifying a test molecule able to modulate the tip growth of cells with cell wall and having an oval or rod shape. More specifically, it concerns a method for screening or identifying a test molecule able to modulate the tip growth of cells with cell wall and having an oval or rod shape comprising:
a) providing a device according to the method of the present invention bearing cells which have been contacted with a test molecule;
b) observing the tip growth of the cells;
c) comparing the tip growth of the cells in presence and in absence of the test molecule; and,
d) selecting the test molecule for which the tip growth is significantly different in presence and in absence of the test molecule, thereby identifying a test molecule able to modulate the tip growth.

The molecules able to modulate the tip growth, in particular inhibit the growth, present an interest for preventing the microorganism growth. Then, they can be used as antibacterial or anti-fungal drugs.

Further aspects and advantages of the present invention will be disclosed in the following experimental section, which should be regarded as illustrative and not limiting the scope of the present application.

### EXAMPLES

### Example 1 : Yeast

### Scope

The inventors present here examples for placing vertically fission yeast cells. Cavities of several dimensions were prepared for two geometries: cylinders as in the patent application WO2010092116, and cones. Cones have a depth of 8 µm, a width of the top of 6 µm and a width of the bottom of 3 µm. Since the diameter of fission yeast is ~ 4 µm, they designed cavities with typical dimensions of 4-5 µm for both geometries. Wells masters were prepared with micro fabrication processes by using masks and DRIE [A CMOS Compatible Ultrasonic Transducer Fabricated With Deep Reactive Ion Etching; Libor Rufer, Christian C. Domingues, Salvador Mir, Valérie Petrini, Jean-Claude Jeannot, and Patrick Delobelle, JOURNAL OF MICROELECTROMECHANICAL SYSTEMS, VOL. 15, NO. 6, p. 1766, DECEMBER 2006]. They were used as molds for preparing final devices by standard soft lithography. Cells were then centrifuged in the cavities and observed as previously reported WO2010092116. The filling percentage was reaching ~ 80% for cones in contrast to ~ 20% for cylinders. This result shows the increased quality of the device with conical cavities for High Content Screening.

### Device reparation

Figure 1 illustrates the design for new cavities. In Figure 2, it is shown the silicon masters that were prepared for cylindrical cones preparation. They were generated with methods described in [A CMOS Compatible Ultrasonic Transducer Fabricated With Deep Reactive Ion Etching; Libor Rufer, Christian C. Domingues, Salvador Mir, Valérie Petrini, Jean-Claude Jeannot, and Patrick Delobelle, JOURNAL OF MICROELECTROMECHANICAL SYSTEMS, VOL. 15, NO. 6, p. 1766, DECEMBER 2006]. Conical cavities are periodically spaced and their shape is perfectly reproducible. The final cavities are prepared by pouring liquid PDMS onto these masters as previously reported (see WO2010092116, references therein); they are allowed to cure at 65°C for 4 hours, before being unpeeled carefully, after plasma treatment. For cylinders the same protocol was used as before.

### Cell insertion

Cells were grown in YE4S in exponentially growing phase and they were treated as before (see WO2010092116 and references therein). Briefly, fission yeasts were centrifuged inside wells, after a short sonication to separate cells. Note that no nitrogen starvation was needed to increase the percentage of filling of the cavities. Samples were then washed thoroughly with fresh medium EMM4S in order to remove cells that had not entered the wells. Cells were then observed under the microscope with a temperature control of the set-up.

### Cell vitality

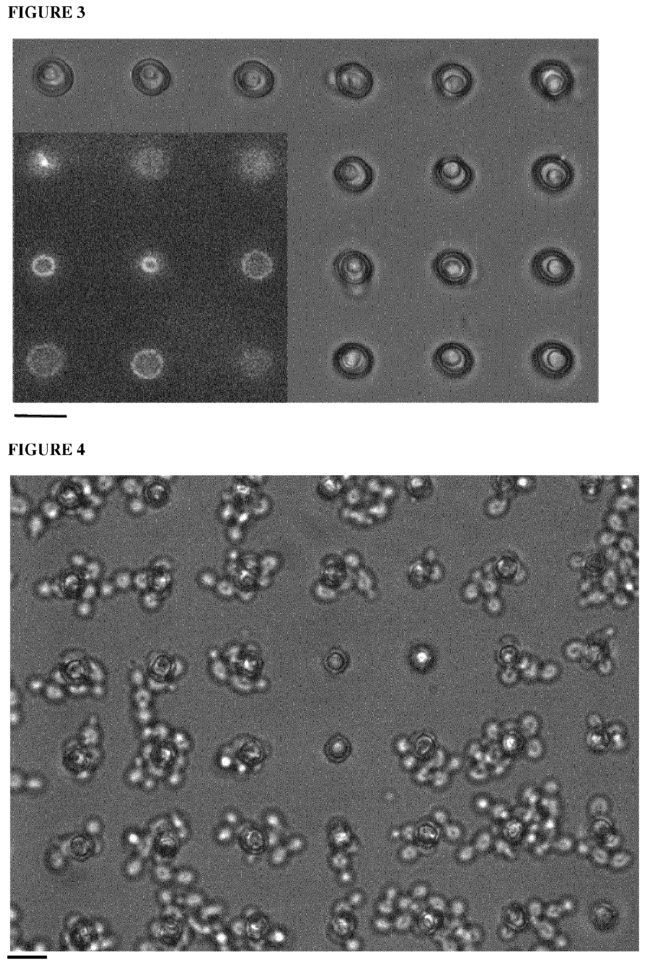

The duration for the cell cycle was unchanged with this protocol. The inventors also checked that the time for cytokinesis was not modified as well. Altogether this shows that the new geometry and shapes of devices do not affect cell growth. This important feature was also observed for another system, *S. cerevisiae* (see Figure 4).

### Percentage of filling

The inventors compared the quality of filling for both types of cavities (see Table 1). Strikingly, cells were filling about 80% of the wells with conical shapes. In contrast only 20% of the cavities were filled when wells had cylindrical shapes. This result suggests that the cells are held tight in the *conical* wells, thereby resisting to the flow of the washing steps.

**Table 1 : Filling percentage depends on the shape. In conical shaped cavities, yeasts are held tightly, as shown by the high filling percentage.**

| ***Cylinders*** | ***Cones*** |
|---|---|
| ***∼20*%** | ***∼80%*** |

### Example 2 : C elegans embryo

### Device suitable to study developing embryos

The device allows the observation of cells in a new configuration. In particular, it makes it possible to standardize cells and to visualize organelles from another perspective, as shown above. For similar reasons, such an approach is very useful for other biological systems. *Caenorhabditis elegans* (C. *elegans*) is a model organism of key importance in developmental biology. It is a worm. Its genetics is easy to manipulate and control. This system has a constant number of cells and it exhibits a perfect organisation of organs, among many advantages. Research on this system has become established worldwide, and key discoveries in biology were made with C. *elegans.*

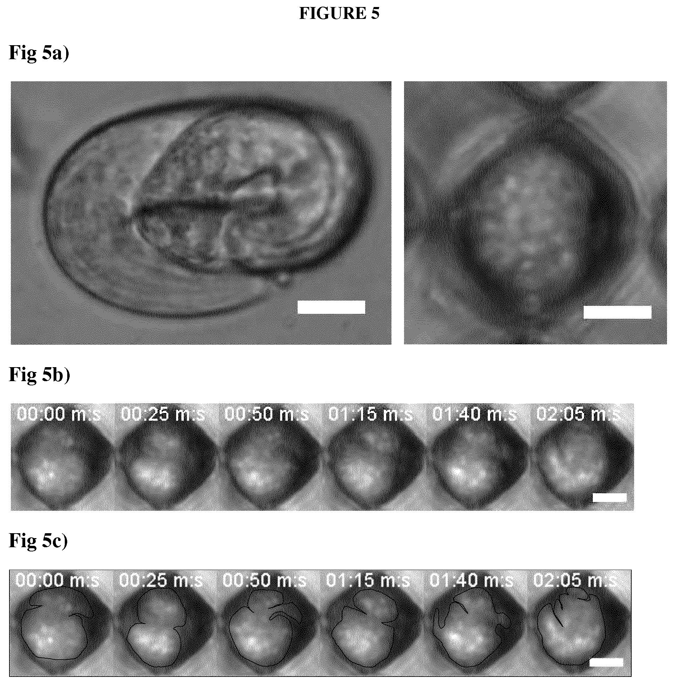

Figure 5a) shows a *C.elegans* embryo in the usual perspective and in the egg cup.

In the time lapse, the characteristic movements during development are visible (Figures 5b) and 5c) with the outline in black).

### Materials and methods

Strains were propagated and handled as described previously in Brenner, S. The genetics of Caenorhabditis elegans. Genetics 77, 71-94 (1974). The C. *elegans* eggs were then centrifuged into the egg cups (5 min, 4000 rpm). The eggs were stored in water and observed with a ×20 objective in a phase contrast inverted microscope (Olympus) equipped with a CCD camera (Hamamatsu). The cavities were made of PDMS, and their diameter was 25 µm; they were spaced by 30 µm (center to center). Time lapse movies were acquired, and the time between images was typically 5 s.

## Claims

1. A method for immobilizing a plurality of cells with rigid cell wall or invertebrate embryos with rigid eggshell on a support of a device bearing a plurality of wells suitable for containing only one single cell or invertebrate embryo, wherein said cells or embryos have an oblong shape, wherein the cells or embryos are immobilized with the same orientation wherein the long axis of the cells or embryos is perpendicular to the support, and wherein :
- the wells have a conical or frustoconical shape;
- the width of the top of the wells is greater than the short axis of the cells or of the embryos to be immobilized in the wells; and
- the width of the bottom of the wells is smaller than the short axis of the cells or of the embryos to be immobilized in the wells;
the method comprising the step of placing said cells or embryos into the wells.

2. The method according to claim 1, wherein the width of wells equal to the short axis of the cells or embryos to be immobilized in the wells is located in the lower half of the wells, preferably in the lower third of the wells.

3. The method according to claim 1 or 2, wherein the angle between the vertical axis of the wells and the side of the wells is between 5 and 45°, preferably between 10 and 30°.

4. The method according to any one of claims 1-3, wherein the depth of the wells is at least 1/3 of the long axis of the cells or embryos to be immobilized in the wells, preferably at least or about 1/2 of the long axis of the cells or embryos to be immobilized in the wells.

5. The method according to any one of claims 1-4, wherein the cells are selected from yeast and bacteria and the embryos are selected from embryos of C. *elegans* or *Drosophila.*

6. The method according to any one of claims 1-4, wherein the cells are yeast and the width of the top of the wells is between 6 to 10 µm and the width of the bottom of the wells is less than 1-2 µm.

7. The method according to any one of claims 1-4, wherein the cells are bacteria and the width of the top of the wells is comprised between 500 nm and 3 µm, and the width of the bottom of the wells is less than 500 nm.

8. The method according to any one of claims 1-4, wherein the embryos are embryos of C. *Elegans* and the width of the top of the wells is comprised between 26 µm and 40 µm, and the width of the bottom of the wells is less than 25 µm.

9. The method according to any one of claims 1-4, wherein the embryos are embryos of *Drosophila* and the width of the top of the wells is comprised between 0.2 mm and 0.3 mm, and the width of the bottom of the wells is less than 0.16 mm.

10. The method according to any one of claims 1-9, wherein the support is suitable for microscopy, for instance a plate of glass, preferably a glass coverslip, with a microfabricated substrate bearing the wells, preferably made of poly(dimethylsiloxane) (PDMS).

11. The method according to any one of claims 1-10, wherein the device further comprises physical barriers separating several groups of wells from each other on the device and, optionally further comprises microfluidic system in order to address fluids to the groups of wells on the device and/or, wherein the device comprises several sets of wells, each set having a different width at the top of the wells.

12. The method according to any one of claims 1-10, wherein the method further comprises a previous step of selecting a suitable device for immobilizing the cells or embryos, in particular based on the cells or embryos size, more particular their the short axis.

13. The method according to any one of claims 1-12, wherein the step of placing the cells or embryos into the wells is carried out by a centrifugation step.

14. The method according to any one of claims 1-13, wherein the method comprises a step of observing the cells or embryos immobilized on the support, in particular in a perpendicular plane to the long axis of cells or of embryos.

15. The method according to claim 14, wherein the step of observing or studying the cells or embryos comprises:
- observing the cytokinetic ring of said cells, and/or
- screening or identifying a test molecule able to modulate the cell division of said cells or the embryogenesis of said embryos, and/or
- observing a plane perpendicular to the long axis of said cells or of said embryos, and/or
- screening or identifying a test molecule able to modulate the tip growth of cells, and/or
- determining the level of expression or activity of proteins in said cells or embryos, and/or
- determining the localization or the interaction of proteins in said cells or embryos, and/or
- observing the structure, localization, shape and/or integrity of organelles, cytoskeleton, DNA, or cytokinetic ring in said cells or embryos, and/or
- observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring in said cells or embryos.

## Patentansprüche

1. Verfahren zur Immobilisierung einer Vielzahl von Zellen mit starrer Zellwand oder von wirbellosen Embryonen mit starrer Eihülle auf einem Träger einer Vorrichtung, die eine Vielzahl von Vertiefungen aufweist, die geeignet sind, eine einzige Zelle oder nur einen wirbellosen Embryo zu enthalten, wobei die Zellen oder die Embryonen eine längliche Ausprägung aufweisen, wobei die Zellen oder die Embryonen mit der gleichen Orientierung immobilisiert sind und wobei die Längsachse der Zellen oder der Embryonen senkrecht zum Träger ausgerichtet sind, und wobei:
- die Vertiefungen eine konische oder frustokonische Form haben;
- die Breite des oberen Teils der Vertiefungen größer ist als der Querschnitt der in den Vertiefungen zu immobilisierenden Zellen oder Embryonen; und
- die Breite des Bodens der Vertiefungen schmaler ist als der Querschnitt der in den Vertiefungen zu immobilisierenden Zellen oder Embryonen;
das Verfahren den Schritt des Platzierens der Zellen oder der Embryonen in die Vertiefungen umfasst.

2. Das Verfahren nach Anspruch 1, wobei die Breite der Vertiefungen, die dem Querschnitt der in den Vertiefungen zu immobilisierenden Zellen oder Embryonen entspricht, in der unteren Hälfte der Vertiefungen, vorzugsweise im unteren Drittel der Vertiefungen liegt.

3. Das Verfahren nach Anspruch 1 oder 2, wobei der Winkel zwischen der vertikalen Achse der Vertiefungen und der Seite der Vertiefungen zwischen 5 und 45°, vorzugsweise zwischen 10 und 30° beträgt.

4. Das Verfahren nach einem der Ansprüche 1 bis 3, wobei die Tiefe der Vertiefungen mindestens ein Drittel der Längsachse der in den Vertiefungen zu immobilisierenden Zellen oder Embryonen beträgt, vorzugsweise mindestens oder ungefähr die Hälfte der Längsachse der in den Vertiefungen zu immobilisierenden Zellen oder Embryonen.

5. Das Verfahren nach einem der Ansprüche 1 bis 4, wobei die Zellen aus Hefezellen oder Bakterien und die Embryonen aus C. elegans oder Drosophila ausgewählt sind.

6. Das Verfahren nach einem der Ansprüche 1 bis 4, wobei die Zellen Hefezellen sind und die Breite des oberen Teils der Vertiefungen zwischen 6 bis 10 µm und die Breite des Bodens der Vertiefungen kleiner als 1 bis 2 µm ist.

7. Das Verfahren nach einem der Ansprüche 1 bis 4, wobei die Zellen Bakterien sind und die Breite des oberen Teils der Vertiefungen zwischen 500 nm und 3 µm umfasst und die Breite des Bodens der Vertiefungen kleiner als 500 nm ist.

8. Das Verfahren nach einem der Ansprüche 1 bis 4, wobei die Embryonen Embryonen von C. elegans sind und die Breite des oberen Teils der Vertiefungen zwischen 26 µm und 40 µm umfasst und die Breite des Bodens der Vertiefungen kleiner als 25 µm ist.

9. Das Verfahren nach einem der Ansprüche 1 bis 4, wobei die Embryonen Embryonen von Drosophila sind und die Breite des oberen Teils der Vertiefungen zwischen 0,2 mm und 0,3 mm umfasst und die Breite des Bodens der Vertiefungen kleiner als 0,16 mm ist.

10. Das Verfahren nach einem der Ansprüche 1 bis 9, wobei der Träger für die Mikroskopie geeignet ist, und beispielsweise eine Glassplatte, vorzugsweise ein Deckglass ist, mit einem mikrofabrizierten Substrat, welches die Vertiefungen aufweist, vorzugsweise aus Poly(dimethylsiloxan) (PDMS).

11. Das Verfahren nach einem der Ansprüche 1 bis 10, wobei die Vorrichtung ferner physische Barrieren umfasst, die mehrere Gruppen von Vertiefungen voneinander auf der Vorrichtung trennen, und ferner gegebenenfalls ein mikrofluides System umfasst, um Flüssigkeiten zu den Gruppen von Vertiefungen auf der Vorrichtung zu bringen, und/oder wobei die Vorrichtung verschiedene Zusammenstellungen von Vertiefungen umfasst, wobei jede Zusammenstellung eine unterschiedliche Breite des oberen Teils der Vertiefungen aufweist.

12. Das Verfahren nach einem der Ansprüche 1 bis 10, wobei das Verfahren ferner einen vorangehenden Schritt zur Auswahl einer geeigneten Vorrichtung zur Immobilisierung der Zellen oder Embryonen umfasst, insbesondere basierend auf der Größe der Zellen oder der Embryonen, speziell auf ihrem Querschnitt.

13. Das Verfahren nach einem der Ansprüche 1 bis 12, wobei der Schritt des Platzierens der Zellen oder der Embryonen in die Vertiefungen mittels eines Zentrifugationsschritts durchgeführt wird.

14. Das Verfahren nach einem der Ansprüche 1 bis 13, wobei das Verfahren einen Beobachtungsschritt der auf dem Träger immobilisierten Zellen oder Embryonen umfasst, insbesondere in senkrechter Ebene zur Längsachse der Zellen oder Embryonen.

15. Das Verfahren nach Anspruch 14, wobei der Beobachtungsschritt oder Untersuchungsschritt der Zellen oder Embryonen umfasst:
- Beobachten des zytokinetischen Rings der Zellen, und/oder
- Durchmustern und Identifizieren eines Testmoleküls mit der Fähigkeit, die Zellteilung der Zellen oder die Embyogenese der Embryonen zu modulieren, und/oder
- Beobachten einer Ebene senkrecht zur Längsachse der Zellen oder Embryonen, und/oder
- Durchmustern und Identifizieren eines Testmoleküls mit der Fähigkeit, das Spitzenwachstum der Zellen zu modulieren, und/oder
- Bestimmen des Expressions- oder Aktivitätsgrads von Proteinen in den Zellen oder Embryonen, und/oder
- Bestimmen der Lokalisierung oder der Interaktion von Proteinen in den Zellen oder Embryonen, und/oder
- Beobachten der Struktur, Lokalisierung, Ausprägung und/oder Intaktheit von Organellen, Zytoskelett, DNA oder zytokinetischem Ring in den Zellen oder Embryonen, und/oder
- Beobachten der Wirkung von Molekülen, Antikörpern, Arzneimitteln oder siRNA auf den Expressionsgrad von Proteinen, den Aktivitätsgrad von Proteinen, der Lokalisierung oder Interaktion von Proteinen, der Struktur, Lokalisierung, Ausprägung und/oder Intaktheit von Organellen, Zytoskelett, DNA oder zytokinetischem Ring, dem Schluss des zytokinetischen Rings in den Zellen oder Embryonen.

## Revendications

1. Méthode d'immobilisation d'une pluralité de cellules ayant une paroi cellulaire rigide ou d'embryons d'invertébrés ayant une coquille rigide sur un support d'un dispositif portant une pluralité de puits adaptés pour ne contenir qu'une seule cellule ou qu'un seul embryon d'invertébré, dans laquelle lesdites cellules ou lesdits embryons ont une forme oblongue,
dans laquelle les cellules ou les embryons sont immobilisés avec la même orientation dans laquelle l'axe long des cellules ou des embryons est perpendiculaire au support, et dans laquelle :
- les puits ont une forme conique ou tronconique ;
- la largeur au sommet des puits est supérieure à l'axe court des cellules ou des embryons qui doivent être immobilisés dans les puits ; et
- la largeur au fond des puits est inférieure à l'axe court des cellules ou des embryons qui doivent être immobilisés dans les puits ;
la méthode comprenant l'étape consistant à placer lesdites cellules ou lesdits embryons dans les puits.

2. Méthode selon la revendication 1, dans laquelle la largeur des puits égale à l'axe court des cellules ou des embryons qui doivent être immobilisés dans les puits se trouve dans la moitié inférieure des puits, de préférence dans le tiers inférieur des puits.

3. Méthode selon la revendication 1 ou 2, dans laquelle l'angle entre l'axe vertical des puits et le bord des puits est compris entre 5 et 45°, de préférence entre 10 et 30°.

4. Méthode selon l'une quelconque des revendications 1 à 3, dans laquelle la profondeur des puits est d'au moins 1/3 de l'axe long des cellules ou des embryons qui doivent être immobilisés dans les puits, de préférence d'au moins 1/2 de l'axe long des cellules ou des embryons qui doivent être immobilisés dans les puits.

5. Méthode selon l'une quelconque des revendications 1 à 4, dans laquelle les cellules sont choisies parmi les levures et les bactéries, et les embryons parmi les embryons de *C*. *elegans* ou de *Drosophila.*

6. Méthode selon l'une quelconque des revendications 1 à 4, dans laquelle les cellules sont des levures et la largeur au sommet des puits est comprise entre 6 et 10 µm et la largeur au fond des puits est inférieure à 1-2 µm.

7. Méthode selon l'une quelconque des revendications 1 à 4, dans laquelle les cellules sont des bactéries et la largeur au sommet des puits est comprise entre 500 nm et 3 µm, et la largeur au fond des puits est inférieure à 500 nm.

8. Méthode selon l'une quelconque des revendications 1 à 4, dans laquelle les embryons sont des embryons de *C*. *elegans* et la largeur au sommet des puits est comprise entre 26 et 40 µm, et la largeur au fond des puits est inférieure à 25 µm.

9. Méthode selon l'une quelconque des revendications 1 à 4, dans laquelle les embryons sont des embryons de *Drosophila* et la largeur au sommet des puits est comprise entre 0,2 et 0,3 mm, et la largeur au fond des puits est inférieure à 0,16 mm.

10. Méthode selon l'une quelconque des revendications 1 à 9, dans laquelle le support se prête à la microscopie, par exemple, une plaque en verre, de préférence une lamelle en verre, à substrat micro-usiné portant les puits, de préférence à base de poly(diméthylsiloxane) (PDMS).

11. Méthode selon l'une quelconque des revendications 1 à 10, dans laquelle le dispositif comprend en outre des barrières physiques séparant plusieurs groupes de puits les uns des autres sur le dispositif et, qui éventuellement comprend en outre un système microfluidique afin de distribuer des fluides aux groupes de puits sur le dispositif et/ou, dans laquelle le dispositif comprend plusieurs ensembles de puits, chaque ensemble ayant une largeur au sommet des puits différente.

12. Méthode selon l'une quelconque des revendications 1 à 10, dans laquelle la méthode comprend une étape préalable consistant à choisir un dispositif adapté pour immobilier les cellules ou les embryons, en particulier en fonction de la taille des cellules ou des embryons, plus particulièrement en fonction de leur axe court.

13. Méthode selon l'une quelconque des revendications 1 à 12, dans laquelle l'étape consistant à placer les cellules ou les embryons dans les puits est mise en oeuvre par une étape de centrifugation.

14. Méthode selon l'une quelconque des revendications 1 à 13, dans laquelle la méthode comprend une étape consistant à observer les cellules ou les embryons immobilisés sur le support, en particulier dans un plan perpendiculaire à l'axe long des cellules ou des embryons.

15. Méthode selon la revendication 14, dans laquelle l'étape consistant à observer ou à étudier les cellules ou les embryons comprend :
- l'observation de l'anneau cytocinétique desdites cellules, et/ou
- le criblage ou l'identification d'une molécule test capable de moduler la division cellulaire desdites cellules ou l'embryogenèse desdits embryons, et/ou
- l'observation d'un plan perpendiculaire à l'axe long desdites cellules ou desdits embryons, et/ou
- le criblage ou l'identification d'une molécule test capable de moduler la croissance apicale des cellules, et/ou
- la détermination du niveau d'expression ou d'activité des protéines dans lesdites cellules ou lesdits embryons, et/ou
- la détermination de la localisation ou de l'interaction des protéines dans lesdites cellules ou lesdits embryons, et/ou
- l'observation de la structure, de la localisation, de la forme et/ou de l'intégrité des organelles, du cytosquelette, de l'ADN, ou de l'anneau cytocinétique dans lesdites cellules ou lesdits embryons, et/ou
- l'observation de l'effet de molécules, d'anticorps, de médicaments, ou de siARN sur le niveau d'expression des protéines, le niveau d'activité des protéines, la localisation ou l'interaction des protéines, la structure, la localisation, la forme et/ou l'intégrité des organelles, du cytosquelette, de l'ADN, ou de l'anneau cytocinétique, la fermeture de l'anneau cytocinétique dans lesdites cellules ou lesdits embryons.
